# EUROPÄISCHE PATENTSCHRIFT

(11) **EP 3 041 525 B1**
(45) Veröffentlichungstag und Bekanntmachung des Hinweises auf die Patenterteilung: **30.10.2019**
(21) Anmeldenummer: 14766125.0
(22) Anmeldetag: 05.09.2014
(51) Int. Cl.: A61M 1/34, A61M 5/44

(54) **MEDIZINISCHE VORRICHTUNG MIT EINER STEUERVORRICHTUNG ZUM BEEINFLUSSEN DES DRUCKS INNERHALB EINES HEIZBEUTELS WÄHREND EINER MEDIZINISCHEN BEHANDLUNG**
MEDICAL DEVICE WITH A CONTROL DEVICE FOR INFLUENCING THE PRESSURE INSIDE A HEATING BAG DURING A MEDICAL TREATMENT
DISPOSITIF MEDICAL AVEC UN DISPOSITIF DE COMMANDE PERMETTANT D'INFLUENCER LA PRESSION A L'INTERIEUR D'UNE POCHE CHAUFFANTE PENDANT UN TRAITEMENT

(30) Priorität: 06.09.2013 DE 102013014751
(43) Veröffentlichungstag der Anmeldung: 13.07.2016
(73) Patentinhaber: Fresenius Medical Care Deutschland GmbH, 61352 Bad Homburg (DE)
(72) Erfinder: KLEWINGHAUS, Jürgen, 61440 Oberursel (DE)
(74) Vertreter: Bobbert & Partner Patentanwälte PartmbB
(86) Internationale Anmeldenummer: PCT/EP2014/068974
(87) Internationale Veröffentlichungsnummer: WO 2015/032914

(56) Entgegenhaltungen:
- EP-A1- 2 366 419
- WO-A1-99/02206
- WO-A1-2005/072666
- US-A1- 2002 147 426
- US-A1- 2002 147 481
- US-A1- 2009 299 273

## Beschreibung

Die vorliegende Erfindung betrifft eine medizinische Vorrichtung gemäß Anspruch 1.

Aus der Praxis der Dialysebehandlung sind Vorrichtungen bekannt, mittels welcher Dialysierflüssigkeit erwärmt wird, bevor diese in den Dialysator oder Blutbehandlungsfilter, in welchem ein Stoffaustausch über eine - üblicherweise semipermeable - Membran zwischen Blut und Dialysierflüssigkeit erfolgt, eingebracht wird. Manche dieser Heizvorrichtungen erfordern ein sicheres Anliegen oder das Einhalten eines Höchstabstandes zwischen der Heizvorrichtung, welche beispielsweise die Form von Heizwendeln hat, und einem von dieser Heizvorrichtung umgebenen Behältnis, welches beispielsweise als Beutel ausgeführt ist, in welchem sich die zu erwärmende Dialysierflüssigkeit befindet. Bei einer Heizvorrichtung dieser Bauart hängt das Erzielen der gewünschten Erwärmung der Dialysierflüssigkeit davon ab, wie gut das Behältnis an der Heizvorrichtung, beispielsweise ausgeführt als Beutelheizung, anliegt (oder umgekehrt). Dies gilt auch für andere Flüssigkeiten, welche in Behältern erwärmt werden.

Aus der US 2002/147426 A1 sind ein Verfahren und ein Gerät/Apparat zur Kontrolle von Infusionen unter Druck und zur Kontrolle der Temperatur von infundierten Flüssigkeiten bekannt. Parameter, welche eine Ultrafiltrationsrate einen Substitutionsfluss betreffen, sind ebenfalls nicht Gegenstand dieses Dokuments.

Aus der WO 2005/072666 A1 ist ein Verfahren und ein Apparat bekannt, welche der Kontrolle der Temperatur infundierter Flüssigkeiten dienen.

Aus der US 2009/299273 A1 betrifft die peritoneale Dialyse. Aus der EP 2 366 419 A1 ist ein Beutelwärmer bekannt.

Aus der US 2002/147481 A1 sind Vorrichtungen zur Erwärmung von Flüssigkeiten bekannt sowie entsprechende Verwendungsverfahren.

Aus der WO 99/02206 A1 sind ein Verfahren und ein System zur Kontrolle des Widerstands eines Schlauchsystems für den Gebrauch in einem Kreislauf für peritoneale Dialyse bekannt.

Eine Aufgabe der vorliegenden Erfindung ist es, eine Vorrichtung vorzuschlagen zum Erzielen oder Sicherstellen eines gewünschten oder erforderlichen Anliegens der Heizvorrichtung an dem die Flüssigkeit führenden Gefäß oder Behältnis, zum Verhindern eines Kollabierens des Gefäßes oder Behältnisses oder zum Verhindern eines Überschreitens eines maximalen Abstandes zwischen Heizvorrichtung und Gefäß im Gebrauch oder bei der Behandlung des Patienten.

Die erfindungsgemäße Aufgabe kann durch eine medizinische Vorrichtung mit den Merkmalen des Anspruchs 1 gelöst werden.

Erfindungsgemäß wird somit eine Vorrichtung zum Ausführen eines Verfahrens zum Steuern oder Kontrollieren des Drucks, welcher während der Behandlung eines Patienten mittels einer Blutbehandlung innerhalb eines Heizbeutels eines zur Behandlung verwendeten Schlauchsystems oder einer angeschlossenen medizinischen Vorrichtung herrscht, vorgeschlagen.

Das Verfahren umfasst ein Bestimmen des innerhalb des Heizbeutels herrschenden Drucks (bzw. seines Wertes). Es umfasst ferner ein Vergleichen des bestimmten Drucks mit einem Referenzdruck (bzw. dessen Wert) und/oder das Ermitteln seiner Lage bezogen auf einen Referenzdruckbereich (bzw. dessen Wertebereich). Zudem umfasst es ein Ändern eines Behandlungsparameters der Behandlung des Patienten und/oder ein Vorschlagen einer Korrektur des Behandlungsparameters für den Fall, dass der Vergleich ergibt, dass der bestimmte Druck, je nach Ziel der Überwachung, unterhalb oder oberhalb des Referenzdrucks und/oder außerhalb des Referenzdruckbereichs liegt.

Die erfindungsgemäße medizinische Vorrichtung weist optional wenigstens ein Schlauchsystem auf oder ist hiermit verbunden. Sie weist in jedem Fall eine Steuer- oder Regelvorrichtung auf, welche konfiguriert ist zum Durchführen des hierin beschriebenen Verfahrens in wenigstens einer der hierin beschriebenen Ausführungsformen des Verfahrens.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale aufweisen.

In manchen erfindungsgemäßen beispielhaften Ausführungsformen ist das Bestimmen des Drucks ein Ermitteln, ein Abschätzen, ein Rückschließen hierauf, oder ein Messen des Drucks.

In gewissen erfindungsgemäßen beispielhaften Ausführungsformen erfolgt das Bestimmen auf direkte oder indirekt Weise.

In manchen erfindungsgemäßen beispielhaften Ausführungsformen erfolgt das Bestimmen des Drucks durch Messen des Filtratdrucks mittels eines Drucksensors. Dieser kann in fluidischem Kontakt mit dem Inhalt des Heizbeutels stehen.

Erfindungsgemäß ist der Parameter der Behandlung ein Substitutionsfluss (oder eine Substitutionsrate) oder eine Ultrafiltrationsrate. Jeder andere Behandlungsparameter, welcher ein Erhöhen des Drucks bewirken kann, ist ebenfalls erfindungsgemäß umfasst.

In manchen beispielhaften erfindungsgemäßen Ausführungsformen wird der Substitutionsfluss soweit reduziert, dass oder bis der Druck wieder unterhalb bzw. oberhalb (d. h. in jedem Fall: auf der gewünschten Seite) des Referenzdrucks, und/oder innerhalb des Referenzdruckbereichs liegt oder gelangt. Ein Referenzdruckbereich kann einen oberen und einen unteren Schwellenwert haben.

In gewissen erfindungsgemäßen beispielhaften Ausführungsformen entspricht der Referenzdruck dem Umgebungsdruck.

In manchen erfindungsgemäßen beispielhaften Ausführungsformen wird der Druck mittels eines Druckaufnehmers bestimmt, welcher in einer Dialysierflüssigkeitsleitung stromabwärts einer Dialysierflüssigkeitspumpe angeordnet ist.

In gewissen erfindungsgemäßen beispielhaften Ausführungsformen ist der Druckaufnehmer stromaufwärts einer Heizvorrichtung, beispielsweise in einer Stichleitung, angeordnet.

In manchen erfindungsgemäßen beispielhaften Ausführungsformen wird der Druck mittels einer Vorrichtung beeinflusst, (mit)bedingt oder verändert. Die Vorrichtung ist ausgewählt aus einer Gruppe, welche wenigstens ein Ventil, eine Drossel und eine Blende aufweist.

Die vorstehend genannte Vorrichtung zum Beeinflussen des Drucks kann stromaufwärts eines Dialysators und stromabwärts der Heizvorrichtung angeordnet sein. Sie kann stromab einer Substituatpumpe angeordnet sein.

Unter einem "Beeinflussen" des Drucks wird in bestimmten erfindungsgemäßen beispielhaften Ausführungsformen beispielsweise ein Senken und/oder Erhöhen des Drucks mittels der Vorrichtung verstanden. Ein solches Beeinflussen kann stattfinden, falls oder wenn der bestimmte Druck unterhalb oder oberhalb des Referenzdrucks oder außerhalb des Referenzdruckbereichs liegt.

In manchen erfindungsgemäßen beispielhaften Ausführungsformen wird der Druck mittels eines Druckaufnehmers bestimmt, welcher in einer Substituatleitung stromabwärts einer Substituatpumpe, vorzugsweise stromaufwärts einer Heizvorrichtung für Substituat, beispielsweise in einer Stichleitung, angeordnet ist.

In gewissen erfindungsgemäßen beispielhaften Ausführungsformen enthält der Heizbeutel Dialysierflüssigkeit und/oder Substituat.

In bestimmten erfindungsgemäßen beispielhaften Ausführungsformen der medizinischen Vorrichtung ist diese als Blutreinigungseinrichtung ausgestaltet, beispielsweise als Dialysiervorrichtung, als Filtrationsvorrichtung, als Diafiltrationsvorrichtung oder als Dialysevorrichtung in jedem anderen, dem Fachmann bekannten Sinne.

Die medizinische Vorrichtung ist in bestimmten erfindungsgemäßen beispielhaften Ausführungsformen eine Dialysiervorrichtung, die im Besonderen zur Anwendung für die kontinuierliche venöse Hämodiafiltration (CVV-HDF) und/oder zur Anwendung für die Akutdialyse konfiguriert ist.

In gewissen erfindungsgemäßen beispielhaften Ausführungsformen weist das Schlauchsystem einen Schlauchadapter auf.

Der Schlauchadapter weist wenigstens einen Verschlussmechanismus zum reversiblen oder vorübergehenden Verschließen eines durchströmbaren Lumens des Schlauchadapters oder des hiermit verbundenen Schlauchabschnitts, und/oder eine Vorrichtung zum Erzeugen oder Verändern eines Strömungswiderstands auf.

Das Schlauchsystem weist wenigstens einen Schlauchadapter auf und/oder ist integral mit einem solchen Schlauchadapter hergestellt oder weist wenigstens einen Verschlussmechanismus zum Verschließen seines durchströmbaren Lumens und/oder wenigstens eine Vorrichtung zum Erzeugen oder Verändern eines Strömungswiderstands auf.

In einigen Ausführungsformen der vorliegenden Erfindung ist die Veränderung des Strömungswiderstandes sprungartig, und/oder aber nicht stetig.

In manchen Ausführungsformen der vorliegenden Erfindung stehen der Schlauchadapter und der Schlauchabschnitt des Schlauchsystems, mit welchem er verbunden ist, in Fluidkommunikation. Der Schlauchabschnitt wird daher bei seinem Gebrauch von derselben Flüssigkeit und/oder stets derselben Flüssigkeitsmenge durchströmt wie der Schlauchabschnitt.

In einigen Ausführungsformen der vorliegenden Erfindung wird der Strömungswiderstand mittels des Schlauchadapters oder eines seiner Bauteile erzeugt und/oder verändert.

In bestimmten Ausführungsformen der vorliegenden Erfindung erfolgt die Veränderung des Strömungswiderstandes innerhalb des durchströmbaren Lumens des Schlauchadapters.

In besonderen Ausführungsformen der Erfindung entspricht die Veränderung des Strömungswiderstandes einem Druckabfall oder bewirkt einen solchen.

In einigen Ausführungsformen der vorliegenden Erfindung verhindert der Verschlussmechanismus in seinem geschlossenen Zustand ein Durchströmen des Schlauchadapters.

In manchen Ausführungsformen der vorliegenden Erfindung verhindert der Verschlussmechanismus in seinem geschlossenen Zustand ein Eintreten von Dialysierflüssigkeit aus einer Quelle für Dialysierflüssigkeit in den Dialysator hinein. In einigen Ausführungsformen der vorliegenden Erfindung verhindert der Verschlussmechanismus in seinem geschlossenen Zustand ein Eintreten von Substituat aus einer Quelle für Substituat in den extrakorporalen Blutkreislauf hinein.

In einigen erfindungsgemäßen Ausführungsformen wird ein Durchströmen des Schlauchadapters infolge des Verschlussmechanismus in Abhängigkeit eines Druckzustands verhindert, der stromauf des Schlauchadapters, und dort insbesondere stromab einer Heizeinrichtung, etwa in Gestalt einer Beutelheizung, herrscht, mittels welcher die durch den Schlauchadapter geführte Flüssigkeit, etwa Dialysierflüssigkeit, erwärmt worden ist.

In manchen erfindungsgemäßen beispielhaften Ausführungsformen verändert oder verhindert die Vorrichtung zum Erzeugen eines Strömungswiderstandes beim bestimmungsgemäßen Gebrauch des Schlauchadapters eine oder jegliche Fluidströmung stromab des Schlauchadapters und/oder stromauf des Schlauchadapters in Abhängigkeit eines Druckzustands, der stromauf des Schlauchadapters herrscht.

In bestimmten Ausführungsformen der vorliegenden Erfindung dienen der Verschlussmechanismus oder die Vorrichtung zum Erzeugen eines Strömungswiderstands dazu, vorbestimmte Druckzustände stromauf des Schlauchadapters auszuschließen. Hierbei handelt es sich insbesondere um Unterdruckzustände oder Druckzustände unterhalb eines vorbestimmten Mindestdrucks.

Ein Bestimmen oder ein Festlegen des vorbestimmten Mindestdrucks kann durch die Auswahl der einzelnen Elemente wie einem Rückschlagventil oder Druckbegrenzungsventil des Schlauchadapters erfolgen.

Die Begriffe Rückschlagventil oder Druckbegrenzungsventil werden in einigen erfindungsgemäßen beispielhaften Ausführungsformen austauschbar verwendet, wo immer der Fachmann dies als technisch sinnvoll erkennt.

In einigen erfindungsgemäßen beispielhaften Ausführungsformen ist der Mindestdruck ein beliebiger Unterdruck. In bestimmten erfindungsgemäßen beispielhaften Ausführungsformen ist der Mindestdruck ein Druck, bei welchem ein Gefäß, ein Behälter oder ein Beutel der konkret verwendeten Beutelheizung zuverlässig nicht kollabiert, wenn dieser Druck im Inneren hiervon herrscht. In gewissen erfindungsgemäßen beispielhaften Ausführungsformen ist der Mindestdruck der Referenzdruck.

In einigen erfindungsgemäßen beispielhaften Ausführungsformen bezieht sich der Mindestdruck auf einen Innendruck des Gefäßes, Behälters oder Beutels der konkret verwendeten Beutelheizung; in anderen erfindungsgemäßen beispielhaften Ausführungsformen bezieht sich der Mindestdruck auf einen Druck, welcher im Dialysierflüssigkeits-Schlauchsystem zwischen Heizvorrichtung und Dialysator herrscht.

In manchen erfindungsgemäßen beispielhaften Ausführungsformen liegen der Schlauchadapter oder Komponenten hiervon nicht in einer Bypassleitung für die Pumpe.

In gewissen erfindungsgemäßen beispielhaften Ausführungsformen weist das Schlauchsystem, vor allem das Dialysierflüssigkeits-Schlauchsystem, keine Bypassleitung für die Pumpe auf.

In einigen erfindungsgemäßen beispielhaften Ausführungsformen sind der Schlauchadapter oder seine Komponenten keine Vorrichtungen zum Begrenzen eines Drucks oder wirken nicht als solche.

In bestimmten erfindungsgemäßen beispielhaften Ausführungsformen sind der Schlauchadapter oder seine Komponenten Vorrichtungen zum Sicherstellen eines Mindestdrucks oder wirken als solche.

So ist beispielsweise ein hierin genanntes Rückschlagventil oder Druckbegrenzungsventil in einigen erfindungsgemäßen beispielhaften Ausführungsformen nicht vorgesehen, um einen Druck zu begrenzen, indem es bei ausreichend hohem Druck öffnet, sondern es schließt vielmehr, wenn der Druck zu niedrig ist.

Das Schlauchsystem weist in bestimmten Ausführungsformen einen Schlauchabschnitt auf, welcher in eine Pumpe, beispielsweise eine okkludierende Schlauchpumpe wie etwa eine Rollenpumpe, einzulegen ist.

In manchen erfindungsgemäßen beispielhaften Ausführungsformen ist der Schlauchadapter, sein Verschlussmechanismus oder seine Vorrichtung zum Erzeugen oder Verändern eines Strömungswiderstandes ein Ventil und/oder eine Drossel und/oder eine Blende, oder weist wenigstens eines der vorgenannten Elemente auf.

Im Falle eines Ventils kann es beispielsweise ein Rückschlagventil oder Druckbegrenzungsventil mit einem definierten oder vorbestimmten Öffnungsdruck sein.

In bestimmten erfindungsgemäßen beispielhaften Ausführungsformen beträgt der Öffnungsdruck des Verschlussmechanismus des Schlauchadapters mindestens 50 mbar und/oder maximal 350 mbar.

Der o. g. Mindestdruck beträgt in manchen erfindungsgemäßen beispielhaften Ausführungsformen 5 mbar oder liegt 5 mbar über einem stromab des Schlauchadapters oder des Verschlussmechanismus herrschenden Drucks.

In einigen erfindungsgemäßen beispielhaften Ausführungsformen bewirkt die Vorrichtung zum Erzeugen oder Verändern eines Strömungswiderstandes eine Veränderung derart, dass im bestimmungsgemäßen Gebrauch des Schlauchadapters über der Vorrichtung oder über dem Schlauchadapter ein Druckunterschied von mindestens 5 mbar und/oder maximal 1000 mbar, bevorzugt von mindestens 50 und/oder maximal 400 mbar, besonders bevorzugt von mindestens 100 mbar und/oder 350 mbar herrscht.

In manchen erfindungsgemäßen beispielhaften Ausführungsformen ist das Schlauchsystem, beispielsweise das Dialysierflüssigkeits-Schlauchsystem, integral oder einstückig mit dem Schlauchadapter hergestellt oder fest - beispielsweise nur unter Zerstörung von diesem lösbar - mit diesem verbunden.

In bestimmten erfindungsgemäßen beispielhaften Ausführungsformen weist das Schlauchsystems, z. B. das Dialysierflüssigkeits-Schlauchsystem oder das Substituat-Schlauchsystem, zusätzlich einen Behälter (beispielsweise einen Sammel- oder einen Vorratsbehälter) mit Dialysierflüssigkeit und/oder Substituat auf oder ist hiermit verbunden. Dieses Fluid ist zum Durchströmen des Schlauchsystems, oder Teilen hiervon, vorgesehen. Zudem kann das Schlauchsystem optional eine Pumpe aufweisen oder kann mit einer Pumpe verbunden sein, die so angeordnet ist, dass sie die vorgenannte Flüssigkeit im Lumen des Schlauchsystems fördern kann. Die Pumpe kann als Verdrängerpumpe, beispielsweise als Rollenpumpe ausgestaltet sein.

Der Behälter kann als ein oder mehrere Beutel ausgestaltet sein. Solch ein Behälter findet sich beispielweise in der US 2005/020959 A1.

In einigen erfindungsgemäßen beispielhaften Ausführungsformen weist das Schlauchsystem eine Heizvorrichtung zum Erwärmen der Flüssigkeit auf oder ist hiermit verbunden oder weist einen Abschnitt auf, welcher vorgesehen ist, im Gebrauch mit einer Heizvorrichtung verbunden zu werden.

Hierbei ist die Heizvorrichtung in gewissen erfindungsgemäßen beispielhaften Ausführungsformen stromauf des Schlauchadapters positioniert.

Dabei kann die Heizvorrichtung von jenem Typ sein, welcher eines gleichbleibenden, positiven, im Inneren des Behälters herrschenden Drucks bedarf, um die gewünschten Heizergebnisse zu erzielen.

In einigen erfindungsgemäßen beispielhaften Ausführungsformen ist die Heizvorrichtung eine Beutelheizung.

In manchen erfindungsgemäßen beispielhaften Ausführungsformen sind das Schlauchsystem oder Teile hiervon, insbesondere das Dialysierflüssigkeits-Schlauchsystem, als Einwegprodukt ausgestaltet.

In einigen erfindungsgemäßen beispielhaften Ausführungsformen ist das Dialysierflüssigkeits-Schlauchsystem ein Dialysierflüssigkeitsschlauch.

In gewissen erfindungsgemäßen beispielhaften Ausführungsformen ist die erfindungsgemäße medizinische Vorrichtung ein Behandlungssystem oder eine Behandlungsvorrichtung, mit einem extrakorporalen Blutkreislauf und/oder einem erfindungsgemäßen Schlauchsystem, oder sie ist hiermit verbunden.

In einigen erfindungsgemäßen Ausgestaltungen ist der Schlauchadapter mit dem Schlauchsystem, beispielsweise dem Dialysierflüssigkeits-Schlauchsystem, stromab einer Heizvorrichtung, insbesondere einer Beutelheizung, verbunden. Dabei ist die Heizvorrichtung ebenfalls mit dem Schlauchsystem verbunden.

In bestimmten erfindungsgemäßen Ausgestaltungen ist der Schlauchadapter mit den Dialysatorkupplungen des Dialysierflüssigkeits-Schlauchsystems verbunden. Der Schlauchadapter kann hierzu entsprechend ausgestaltet sein, um beispielsweise mit der oder den Dialysatorkupplungen per Steckverbindung, per Steck-Schraubverbindung oder dergleichen verbunden zu werden, insbesondere ohne Zuhilfenahme von Werkzeug oder weiteren Verbindungselementen.

Unter dem Begriff Schlauch"adapter", wie hierin verwendet, ist nicht etwa einschränkend zu verstehen, dass der Schlauchadapter etwas miteinander verbindbar machen soll, was nicht auch ohne ihn verbunden werden kann. Der Begriff umfasst vielmehr auch ein Zwischenelement oder Verbindungselement, welches verwendet werden soll, um zwei Schlauchabschnitte eines Schlauchsystems oder einen Schlauchabschnitt mit einem Dialysator in Fluidkommunikation miteinander, insbesondere unmittelbar, zu verbinden.

In bestimmten erfindungsgemäßen Ausgestaltungen besteht der Schlauchadapter aus einem Verschlussmechanismus, der hier exemplarisch als Rückschlagventil oder Ventil ausgestaltet ist, zwei Schlauchanschlüssen sowie zwei - optional vorgesehenen und vorzugsweise kurzen - Schlauchabschnitten des Schlauchadapters. Der Schlauchadapter kann weitere Komponenten aufweisen.

Die einzelnen Komponenten des Schlauchadapters sind in manchen erfindungsgemäßen Ausführungsbeispielen fest miteinander verbunden, beispielsweise mittels Kleben oder Ultraschallverschweißen. Die Komponenten können jedoch auch wieder lösbar miteinander verbunden sein. Letzteres hätte den Vorteil, dass der Schlauchadapter auch noch am Ort seines Gebrauchs mit verschieden großen Schlauchanschlüssen vorgesehen werden kann, welche in oder auf entsprechende Schlauchanschlüsse anderer Schlauchsysteme passen.

Zum Verbinden des Schlauchadapters werden dessen Schlauchanschlüsse mit Schlauchkupplungen weiterer Abschnitte des Schlauchsystems verbunden. Der Schlauchanschluss kann als erstes Kupplungsteil, hier als sogenanntes "Vaterteil" (auch als Stutzen oder männlicher Verbinder) bezeichnet ausgeführt sein, der Schlauchanschluss als sogenanntes "Mutterteil" (auch als weiblicher Verbinder) bezeichnet sein. Das Mutterteil der Kupplung ist in einigen Ausführungsbeispielen baugleich mit einem entsprechenden Schlauchanschluss des Schlauchabschnitts.

Die beiden Schlauchanschlüsse werden als Kupplung zusammengesteckt, so dass das Schlauchsystem hierdurch um die freiliegende Länge des Schlauchadapters verlängert wird.

Das Ventil des Schlauchadapters öffnet sich - beispielsweise gegen eine Federkraft - dann, wenn stromauf des Ventils ein Mindestdruck anliegt.

In manchen erfindungsgemäßen beispielhaften Ausführungsformen werden die Dialysierflüssigkeitspumpe und die Substituatpumpe aus einem gemeinsamen Behälter oder einer gemeinsamen Quelle versorgt. Dies geschieht in einigen erfindungsgemäßen beispielhaften Ausführungsformen mittels einer gemeinsamen Leitung.

In bestimmten erfindungsgemäßen beispielhaften Ausführungsformen werden die Dialysierflüssigkeitspumpe und die Substituatpumpe aus getrennten Behältern oder Quellen versorgt.

In manchen erfindungsgemäßen beispielhaften Ausführungsformen werden das aus Beuteln oder Quellen an die Dialysierflüssigkeitspumpe und die Substituatpumpe zugeführte Fluid zunächst mittels eines Y-Stücks vereint, oder die aus den beiden Beuteln oder Quellen stammende Fluide werden jeweils durch ein und dasselbe Y-Stück geführt, bevor das Fluid oder die Mischung der Fluide über den gemeinsamen Schenkel des Y-Stücks in die Zuleitung(en) eingespeist wird.

In bestimmten erfindungsgemäßen beispielhaften Ausführungsformen sind die Zuleitungen zum Zuführen von Fluid zur Dialysierflüssigkeitspumpe und zur Substituatpumpe mittels einer Verbindungsleitung in Fluidkommunikation miteinander verbunden.

In manchen erfindungsgemäßen beispielhaften Ausführungsformen mündet das Y-Stück oder die gemeinsame Leitung in die Verbindungsleitung.

Zur Ausgestaltung und Funktion des Schlauchadapters wird auf die Offenlegungsschrift zur Patentanmeldung 10 2012 004 673.1, welche am 12.03.2012 für die Anmelderin der vorliegenden Anmeldung beim Deutschen Patent- und Markenamt (DPMA) eingereicht wurde, verwiesen.

In gewissen erfindungsgemäßen beispielhaften Ausführungsformen weist die erfindungsgemäße medizinische Vorrichtung alle zum Ausführen des hierin beschriebenen Verfahrens erforderlichen Vorrichtungen, Bauteile oder Komponenten auf oder steht hiermit in Signalverbindung. So ist zur Ausführung eines jeden Verfahrensschritts eine, vorzugsweise zum Verfahrensschritt gleichnamige, Vorrichtung vorgesehen, welche zum Ausführen des jeweiligen Verfahrensschritts wenigstens geeignet, entsprechend verbunden und/oder konfiguriert ist. Insbesondere kann die medizinische Vorrichtung eine Vorrichtung zum Bestimmen des Drucks (beispielsweise einen Druckaufnehmer), eine Vorrichtung zum Vergleichen des bestimmten Drucks mit einem Referenzdruck oder Ermitteln seiner Lage bezogen auf einen Referenzdruckbereich (beispielsweise eine Vergleichsvorrichtung) und eine Vorrichtung zum Ändern wenigstens eines Behandlungsparameters der Behandlung des Patienten oder Vorschlagen einer Korrektur oder Änderung des wenigstens einen Behandlungsparameters, falls der bestimmte Druck unterhalb bzw. oberhalb des Referenzdrucks oder außerhalb des Referenzdruckbereichs liegt (beispielsweise eine Korrekturvorrichtung), aufweisen.

Erfindungsgemäß werden in manchen beispielhaften Ausführungsformen somit über den im Heizbeutel herrschenden Druck gewonnene Informationen genutzt, um einen oder mehrere vom Anwender, also dem medizinischen Personal, zur Behandlung des Patienten eingestellten oder ausgewählten Behandlungsparameter gezielt (mit Rückkopplung) zu steuern oder (ohne Rückkopplung) zu regeln. Dies kann jeweils automatisch erfolgen. Alternativ kann dem Anwender ein Vorschlag unterbreitet werden, wie einer oder mehrere der Behandlungsparameter verändert werden könnten. Der Anwender kann mittels Alarm auf den Vorschlag aufmerksam gemacht werden, welchen er beispielsweise auf einem Display angezeigt bekommt. Der Anwender kann den Vorschlag annehmen, ablehnen, oder abändern und anschließend annehmen. Zu den o. g. Parametern zählen wenigstens der Substitutionsfluss sowie die (Ultra-)Filtrationsrate.

In manchen erfindungsgemäßen, beispielhaften Ausführungsformen weist die Heizvorrichtung keine Sensorik zur Überwachung und/oder Feststellung der Verformung und/oder der Position des Heizbeutels, insbesondere keinen mechanischen und/oder optischen Sensor, beispielsweise keinen Druckschalter und/oder kein verschiebbares Element, zu diesem Zweck auf.

In einigen erfindungsgemäßen, beispielhaften Ausführungsformen weist das Schlauchsystem keinen Schlauchadapter auf.

Die erfindungsgemäße medizinische Vorrichtung kann bei der Blutbehandlung des Patienten mit einem Schlauchsystem verbunden sein oder ein solches aufweisen. Das Schlauchsystem weist wenigstens einen Behälter mit einer Flüssigkeit, beispielsweise Dialysierflüssigkeit oder Substituat auf, welche jeweils zum Durchströmen des Schlauchsystems vorgesehen ist. Ferner weist es wenigstens eine Pumpe auf, welche zum Fördern der Flüssigkeit, beispielsweise Dialysierflüssigkeit oder Substituat, in dem Schlauchsystem angeordnet ist, oder ist hiermit jeweils verbunden oder zur Verbindung hiermit vorgesehen. Des Weiteren weist das Schlauchsystem eine Heizvorrichtung zum Erwärmen der Flüssigkeit auf oder ist hiermit verbunden. Die Heizvorrichtung weist einen Heizbeutel mit Flüssigkeit, wie Dialysierflüssigkeit oder Substituat, auf oder ist als ein solcher ausgestaltet.

Manche oder alle erfindungsgemäßen beispielhaften Ausführungsformen können einen, mehrere oder alle der oben und/oder im Folgenden genannten Vorteile aufweisen.

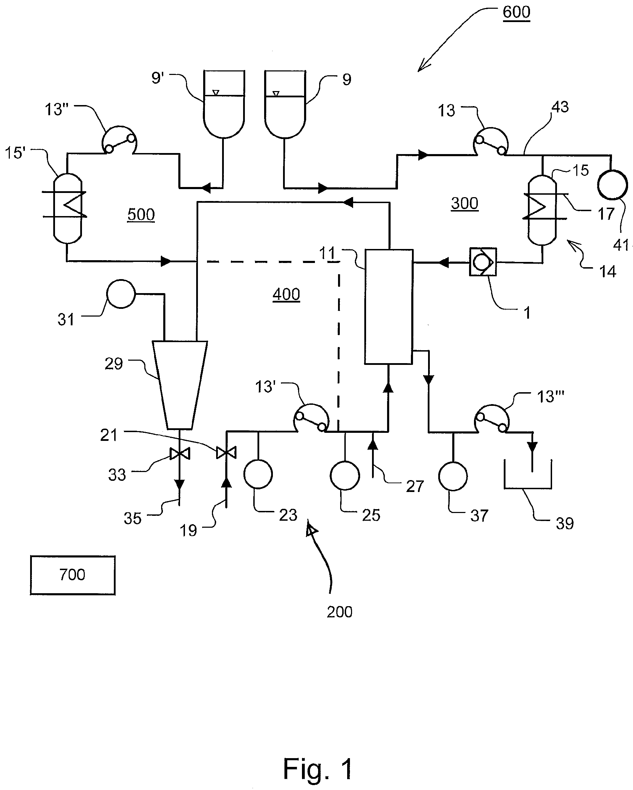

Eine alternative Anordnung, beispielsweise im Dialysierflüssigkeits-Schlauchsystem, wäre eine Pumpe, beispielsweise eine okkludierende Pumpe (z. B. eine Rollenpumpe), stromab der Heizvorrichtung. Die Funktion des Schlauchadapters mit beispielsweise einem Rückschlagventil oder Druckbegrenzungsventil würde dann, zumindest teilweise, von der okkludierenden Pumpe übernommen werden; diese könnte das Auftreten eines Unterdrucks zwischen Heizvorrichtung und Dialysator durch ihre okkludierende Wirkung verhindern. Eine erfindungsgemäße Anordnung mit einem Dialysierflüssigkeits-Schlauchsystem, in dem die Heizvorrichtung, beispielsweise eine Beutelheizung, stromab der Pumpe angeordnet ist und der Schlauchadapter stromab der Heizvorrichtung angeordnet ist, weist die folgenden Vorteile gegenüber der genannten alternativen Lösung auf: Ein Unterdruck in der Heizvorrichtung ist nicht nur durch den Dialysator verursacht möglich (siehe Beschreibung zu Fig. 1: ein hoher TMP im Dialysator kann verschiedene Ursachen haben), sondern auch durch ein Saugen der okkludierenden Pumpe, wie dies bei Rollenpumpen regelmäßig auftritt. Auch durch die Saugwirkung der stromab der Heizvorrichtung angeordneten okkludierenden Pumpe kann der unerwünschte Effekt des Unterdrucks in der Heizvorrichtung auftreten.

Weiterhin werden mit der erfindungsgemäßen Anordnung mögliche Bilanzierfehler vermieden, da die Heizvorrichtung, vor allem die Beutelheizung, zwar als zusätzlicher Volumenspeicher anzusehen ist, in der Regel aber nicht im Bilanziersystem berücksichtigt ist. Eine Veränderung aufgrund Unterdrucks und Kollabierens des Gefäßes der Heizvorrichtung, vor allem der Beutelheizung, wie dies bei der o. g. alternativen Lösung auftreten kann, kann vorteilhaft mit einem Schlauchadapter vermieden werden.

Weiterhin kann erfindungsgemäß vorteilhaft ein Erwärmen der Dialysierflüssigkeit, ohne dass die Dialysierflüssigkeit unmittelbar gefördert wird, wie dies bei einer alternativen Anordnung möglich wäre, bei bestimmten Bicarbonat-haltigen Lösungen vermieden werden. Bei einer derartigen Erwärmung kann die Qualität der Dialysierflüssigkeit beeinträchtigt werden. Es kann zu Ausfällungen der Dialysierflüssigkeit kommen. Aufgrund der erfindungsgemäßen Anordnung der Pumpe stromauf der Heizvorrichtung kann eine ungewollte Abkühlung des Dialysats vor dem Eintritt in den Dialysator vorteilhaft vermieden werden. Durch eine ungewollte Abkühlung könnte es zu schwer kalkulierbaren Temperaturabweichungen kommen. Diese können erfindungsgemäß unter Erzielen der hiermit einhergehenden Vorteile vermieden werden.

Zum Erkennen eines Unterdrucks in der Heizvorrichtung, beispielsweise einer Beutelheizung, mit dem Ziel, einen solchen zu vermeiden, bedarf es bei der erfindungsgemäßen Lösung zwischen der Heizvorrichtung und dem Dialysator keiner Überwachung des Filtratdrucks, also des Dialysatflüssigkeitsdrucks im Dialysator unmittelbar an der Membran, oder eines anderen Drucks auf einen geeigneten Mindestwert hin. So liegt etwa bei einer Anordnung zur kontinuierlich venösen Hämodiafiltration (CVV-HDF) im Wesentlichen kein relevanter Flusswiderstand zwischen Filtratdrucküberwachung und der Heizvorrichtung vor. Zwar wäre es nach einer Korrektur um einen hydrostatischen Druckunterschied möglich, einen Mindestfiltratdruck zu definieren, bei dem sichergestellt ist, dass der Druck in der Heizvorrichtung ausreichend hoch ist, um ein Kollabieren eines Beutels zu verhindern. Bei Unterschreiten dieses Mindestfiltratdrucks könnte beispielsweise die Flussrate der Pumpe gedrosselt werden. Hierzu müsste allerdings ein Steuer- oder Regelkreis aufgebaut werden, was mit Kosten und Aufwand, ferner mit Wartung, Eichung und dergleichen verbunden ist. Dies ist erfindungsgemäß nicht erforderlich.

Ferner birgt eine solche Lösung die Gefahr, dass es in der Folge zu Fehlalarmen innerhalb des erlaubten Flussratenbereichs der Pumpen kommen könnte und die Verfügbarkeit bestimmter Behandlungsparameter eingeschränkt ist. Es könnte zu nicht interpretierbaren Warnungen (leerlaufender Heizbeutel, Bilanzwarnungen, Heizungsalarme) kommen. Durch den Einsatz des Schlauchadapters kann vorteilhaft auf eine Überwachung eines Mindestfiltratdrucks verzichtet werden.

Ein weiterer Vorteil besteht darin, dass der Innendruck eines Beutels der Heizvorrichtung mittels des Schlauchadapters um einen erforderlichen Wert zwischen 5 und 1000 mbar, bevorzugt zwischen 50 und 400 mbar, besonders bevorzugt zwischen 100 und 350 mbar angehoben werden kann. Dieser Wert wird durch die Auswahl einer handelsüblichen, oben genannten Vorrichtung, wie einem Ventil, anhand des Öffnungsdrucks in Flussrichtung einstellbar oder auswählbar.

Die erfindungsgemäß erzielbare Erhöhung des Drucks wirkt einem unerwünschten Ausgasen der Lösung bei Kontakt mit der heißen Heizoberfläche, wie dies bei niedrigem Druck geschehen kann, vorteilhaft entgegen oder verhindert dies im Wesentlichen. Somit kann eine pH-Verschiebung durch das Ausgasen, vor allem wenn CO₂ ausgegast wird, und somit das unerwünschte Ausfällen von Calciumcarbonat und anderen Verbindungen aus der strömenden Lösung vermieden werden.

Vorteilhaft ist auch, dass der Schlauchadapter als Nachrüstteil auch erst nach Erkennen des Problems einer kollabierenden Heizvorrichtung oder Heizbeutels einsetzbar ist. Damit kann eine teure und aufwendige Integration beispielsweise eines Rückschlagventils oder Druckbegrenzungsventils in jedes einzelne der produzierten Dialysat-Schlauchsysteme vermieden werden.

Die vorliegende Erfindung wird im Folgenden anhand der beigefügten Zeichnungen, in welchen identische Bezugszeichen gleiche oder ähnliche Bauteile bezeichnen, exemplarisch erläutert. In den zum Teil stark vereinfachten Figuren gilt:
- **Fig. 1**: zeigt schematisch eine erfindungsgemäße medizinische Vorrichtung für die Dialyse mit oder in einem extrakorporalen Blutkreislauf und einem Schlauchsystem in einer ersten Ausführungsform;
- **Fig. 2**: zeigt schematisch eine erfindungsgemäße medizinische Vorrichtung für die Dialyse mit oder in einem extrakorporalen Blutkreislauf und einem Schlauchsystem in einer zweiten Ausführungsform;
- **Fig. 3**: zeigt schematisch eine erfindungsgemäße medizinische Vorrichtung für die Dialyse mit oder in einem extrakorporalen Blutkreislauf und einem Schlauchsystem in einer dritten Ausführungsform;
- **Fig. 4**: zeigt schematisch eine erfindungsgemäße medizinische Vorrichtung für die Dialyse mit oder in einem extrakorporalen Blutkreislauf und einem Schlauchsystem in einer vierten Ausführungsform;
- **Fig. 5**: zeigt schematisch stark vereinfacht eine erste erfindungsgemäße Weiterbildung eines Ausschnitts der medizinischen Vorrichtung; und
- **Fig. 6**: zeigt schematisch stark vereinfacht eine zweite erfindungsgemäße Weiterbildung eines Ausschnitts der medizinischen Vorrichtung.

**Fig. 1** zeigt ein Schlauchsystem 200 mit einem Dialysierflüssigkeits-Schlauchsystem 300 und einem Substituat-Schlauchsystem 500 in einer ersten Ausführungsform als Teil einer erfindungsgemäßen medizinischen Vorrichtung 600, welche hier rein exemplarisch ein Behandlungssystem für eine Dialyse ist. Die medizinische Vorrichtung 600 weist weiterhin einen Blutkreislauf 400 (hier als Ausschnitt dargestellt) auf, ferner einen Dialysator 11, der von zwei Fluidsystemen (Blut und Dialysierflüssigkeit) zum Stoffaustausch durchströmt wird.

Das Dialysierflüssigkeits-Schlauchsystem 300, das als Einweg-Schlauchsystem ("Disposable") ausgeführt sein kann, wird mit einer Dialysierflüssigkeit aus einem Beutel 9 befüllt. Die Dialysierflüssigkeit wird mittels einer hier nur exemplarisch als Rollenpumpe ausgestalteten Pumpe 13 im Dialysierflüssigkeits-Schlauchsystem 300 gefördert. Dabei kann die Dialysierflüssigkeit aus dem Beutel 9 mittels Schwerkraft und/oder durch Ansaugen mittels der Pumpe 13 zwischen dem Beutel 9 und einer stromab der Pumpe 13, also auf der Druckseite der Pumpe 13, gelegenen Heizvorrichtung 14 fließen. Die in den Figuren gezeigte Heizvorrichtung 14 ist exemplarisch als eine Vorrichtung ausgestaltet, welche wenigstens einen Heizbeutel 15 und Heizstäbe oder Heizwendeln 17 aufweist, weshalb sie hierin auch als Beutelheizung bezeichnet wird. Im Heizbeutel 15 wird die Dialysierflüssigkeit erwärmt. Zur Gewährleistung des Wärmeübergangs von der Heizvorrichtung 14 oder den Heizwendeln 17 auf den Heizbeutel 15 ist es hilfreich, ggf. gar notwendig, dass in dem Heizbeutel 15 wenigstens ein Referenzdruck, in der Regel oder vorzugsweise ein positiver, vorzugsweise festlegbarer, Innendruck der Dialysierflüssigkeit gegenüber dem Umgebungsdruck, herrscht. Auf diese Weise legt sich die Beutelwand des Heizbeutels 15 an die Heizwendeln 17 an, was den Wärmeübergang ermöglicht oder begünstigt. Anders ausgedrückt wird hier exemplarisch mittels eines positiven Innendrucks ein Kollabieren des Heizbeutels 15 und eine Verschlechterung oder Unterbrechung des Wärmeübergangs verhindert.

Weiter stromab der Pumpe oder Dialysierflüssigkeitspumpe 13 und stromab der Heizvorrichtung 14 ist rein optional ein Ventil 1 angeordnet. In diesem Ausführungsbeispiel ist das Ventil 1 bereits in das Schlauchsystem 300 integriert, Fig. 1 zeigt daher keinen Schlauchadapter, was ebenfalls von der vorliegenden Erfindung umfasst ist. Das wie hier integrierte Ventil 1 bedarf daher keiner Schlauchkupplungen. Alternativ kann das Ventil 1 jedoch auch als Teil eines Schlauchadapters an das Schlauchsystem 200 angeschlossen werden.

Das Ventil 1 ist derart in dem Schlauchsystem 300 angeordnet, dass bei einem zu niedrigen Druck stromauf des Ventils 1, also zwischen Ventil 1 und Heizvorrichtung 14 oder Heizbeutel 15, das Ventil 1 schließt. Mit diesem Schließen wird auch verhindert, dass sich ein stromab des Ventils 1 herrschender zu niedriger Druck stromaufwärts des Ventils 1 bis in den Heizbeutel 15 fortsetzt und dort möglicherweise zu einem negativen Innendruck (gegenüber dem Umgebungsdruck) oder einem unerwünscht niedrigen Innendruck führt. Wie weiter oben bereits diskutiert wurde, ist ein positiver Innendruck im Heizbeutel 15 hilfreich, um den optimalen, den gewünschten oder den erwarteten Wärmeübergang zu gewährleisten.

Der Öffnungsdruck des Ventils 1 liegt in manchen erfindungsgemäßen beispielhaften Ausführungsformen zwischen 5 und 1000 mbar, bevorzugt zwischen 50 und 400 mbar, besonders bevorzugt zwischen 100 und 350 mbar. Dies bedeutet, dass der Innendruck des Heizbeutels 15 mindestens diesen Wert betragen muss oder mindestens um diesen Wert höher sein muss als ein Druck stromab des Ventils. Damit das Ventil 1 öffnet, lässt man die in dem Schlauchabschnitt zwischen Heizbeutel 15 und Ventil 1 auftretenden Strömungsverluste außer Betracht. Anders ausgedrückt muss der Druck stromauf des Ventils 1 mindestens diesen Wert (zwischen 5 und 1000 mbar, bevorzugt zwischen 50 und 400 mbar, besonders bevorzugt zwischen 100 und 350 mbar) betragen oder um diesen Wert höher liegen, um den Öffnungsdruck des Ventils 1 zu überwinden.

Ein niedriger Druck stromab des Ventils 1, und damit ein "Leersaugen" der Dialysierflüssigkeit weiter stromauf bis hin zum Heizbeutel 15, kann in der Praxis verschiedene Ursachen haben.

Die medizinische Vorrichtung 600 (auch als Behandlungssystem bezeichnet) ist rein exemplarisch für die Dialyse, in diesem Ausführungsbeispiel speziell für die kontinuierliche venovenöse Hämodiafiltration (Kombination von Hämofiltration und Hämodialyse), abgekürzt CVV-HDF, ausgestaltet.

Dem Patienten wird mittels eines arteriellen Anschlusses 19 einer arteriellen Leitung des extrakorporalen Blutkreislaufs 400 Blut entnommen. Stromab des arteriellen Anschlusses 19 ist ein Absperrhahn 21 angeordnet. Wiederum stromab hiervon wird der arterielle Druck mittels eines Drucksensors 23 gemessen, noch weiter stromab ist eine Blutpumpe 13' angeordnet. Zwischen der Blutpumpe 13' und dem Anschluss der arteriellen Leitung an den Dialysator 11 wird der Hämofiltrationsdruck oder Vorfilterdruck mittels eines Drucksensors 25 gemessen. Stromab dieses Drucksensors 25 wird dem Blut an einer Zugabestelle 27 Heparin zur Gerinnungshemmung zugegeben.

Im Dialysator 11 findet der Stoffaustausch mit der Dialysierflüssigkeit des Dialysierflüssigkeits-Schlauchsystems 300, welches den Dialysator 11 als Dialysat verlässt, statt. Dies wird weiter unten näher beschrieben.

Stromab des Dialysators 11 fließt das Blut in eine venöse Tropfkammer 29, in der mittels eines Drucksensors 31 der venöse Druck gemessen wird. Stromab hiervon ist ein Absperrhahn 33 angeordnet. Das Blut wird mittels eines venösen Anschlusses 35 in das Gefäßsystem des Patienten zurückgeführt.

Das Substituat-Schlauchsystem 500 dient dazu, dem Patienten einen Teil des bei der Behandlung entzogenen Flüssigkeitsvolumens, welches dem Blut durch die Filtration oder Ultrafiltration im Dialysator 11 entzogen wurde, zu substituieren. Hierzu wird Substituat-Flüssigkeit aus einem Beutel 9' entnommen. Das Substituat wird im Substituat-Schlauchsystem 500 mittels einer Substituatpumpe 13" in einen Heizbeutel 15' gefördert, dort erwärmt und anschließend dem Blutkreislauf 400 zugeführt.

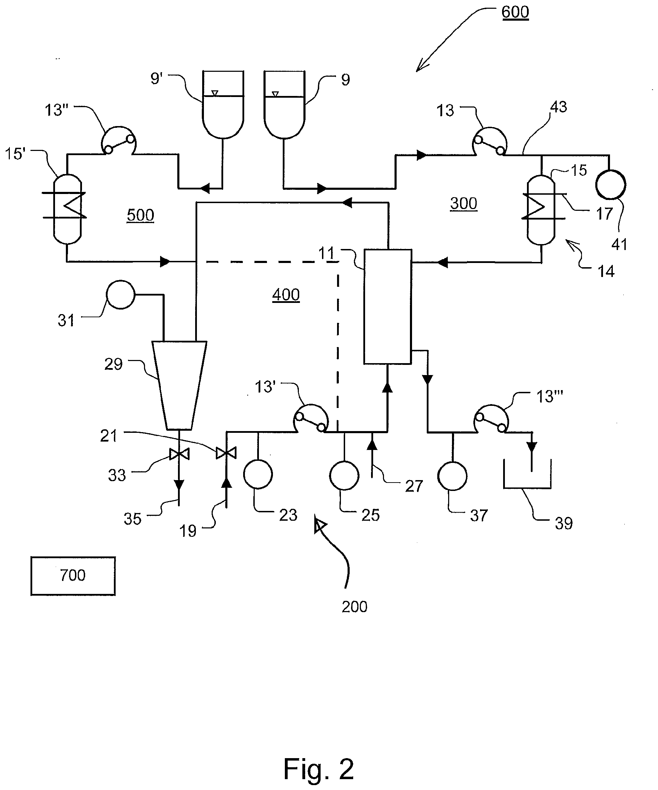

Das Dialysierflüssigkeits-Schlauchsystem 300 stromauf des Dialysators 11 wurde bereits zu Fig. 2 eingehend erläutert. Stromab des Dialysators 11 wird der Filtratdruck mittels eines Drucksensors 37 gemessen, weiter stromab wird das Dialysat gemeinsam mit dem Filtrat mittels einer Pumpe 13"', auch als Dialysat- oder Filtratpumpe bezeichnet, in einen Auffangbehälter 39 gefördert oder verworfen.

Nachfolgend werden in der Praxis auftretende Ursachen eines niedrigen Drucks stromab des Ventils 1 diskutiert.

Ein niedriger Druck (gegenüber dem Umgebungsdruck) stromab des Ventils 1, welcher zu einem "Leersaugen" des Heizbeutels 15 führen könnte, wäre nicht das Ventil 1 vorgesehen, kann beispielsweise durch Ablagerungen auf der Filtermembran des Dialysators 11 (auf der Membranseite des Blutkreislaufs 400; z. B. durch beginnendes "Clotting" des Blutes) bedingt sein. Dies führt zu einer Verringerung der Permeabilität der Filtermembran im Dialysator und damit zu einem Anstieg des Transmembrandrucks TMP (TransMembrane Pressure).

Unabhängig von diesem Phänomen kann die Verwendung von Filtermembranen mit geringer Permeabilität (es treten die gleichen Effekte wie bei Ablagerungen auf der Membran auf) zu diesem Problem des niedrigen Drucks auf der Dialysatseite des Dialysators 11 führen. Eine geringe Permeabilität führt daher zu einem hohen TMP zum Erreichen eines gewünschten bzw. geforderten Stoffaustauschs im Dialysator 11. Die Verwendung des Dialysierflüssigkeits-Schlauchsystems 300 kann daher bei Verwendung von Filtermembranen mit geringer Permeabilität vorteilhaft dazu genutzt werden, einen hohen Unterdruck im Heizbeutel 15 zu vermeiden und damit einen optimalen, gewünschten oder den erwarteten Wärmeübergang vom Heizungsgehäuse auf den Heizbeutel 15 zu gewährleisten.

Ein möglicherweise für die Belange des Heizbeutels zu niedriger Druck auf der Dialysatseite tritt besonders bei Dialyse-Behandlungen auf, die mittels der kontinuierlichen venovenösen Hämodiafiltration (CVV-HDF) durchgeführt werden. Denn hier muss zusätzlich zu der Flüssigkeitsmenge, die dem Patienten mittels des Dialysators 11 entzogen werden soll, auch noch das maschinenseitig zugegebene Substitutionsvolumen durch das Substituat-Schlauchsystem 500 über die Filtermembran des Dialysators 11 aus dem Patientenblut entzogen werden. Dies erfordert hohe Filtrationsflussraten und ein entsprechend hohes Druckgefälle über die Filtermembran, d.h. einen hohen Transmembrandruck TMP.

Der Druckbezugspunkt für den Bereich um die Filtermembran des Dialysators 11 liegt im Bereich des venösen Anschlusses 35. Der Druckbezugspunkt für die Dialysatseite ist der Drucksensor 37. Von dort kann der Druck auf die Dialysatseite der Filtermembran (im Dialysierflüssigkeit-Schlauchsystem 300) rückverfolgt werden, indem strömungsbedingte Druckabfälle und hydrostatische Druckunterschiede berücksichtigt werden. Bei hinreichend hohem TMP ist so nachvollziehbar, dass es zu einem Druck im Heizungsbeutel 15 unterhalb des Umgebungsluftdrucks und damit zu einem Kollabieren des Heizbeutels 15 kommen kann, wenn diesem nicht, beispielsweise durch das Verwenden des Schlauchadapters oder des Dialysierflüssigkeits-Schlauchsystems 300, entgegen gewirkt wird.

Eine Steuer- oder Regelungsvorrichtung 700 ist in den Figuren rein schematisch angedeutet. Sie ist in der Praxis mit den entsprechenden Komponenten wie Druckaufnehmern, Ultrafiltrationspumpe oder Substituatpumpe in Signalkommunikation verbunden.

Fig. 1 zeigt eine beispielhafte Anordnung, bei welcher das Ventil 1 vorgesehen ist. Bei dieser Anordnung kann - als eine von mehreren Möglichkeiten zur Druckbestimmung - der Druck mittels eines optional vorgesehenen Druckaufnehmers 41 bestimmt werden, welcher rein exemplarisch in einer Dialysierflüssigkeitsleitung 43 liegt.

Ist das Ventil 1 nicht vorgesehen, so bietet sich auch eine Druckbestimmung mittels des Drucksensors 37 an.

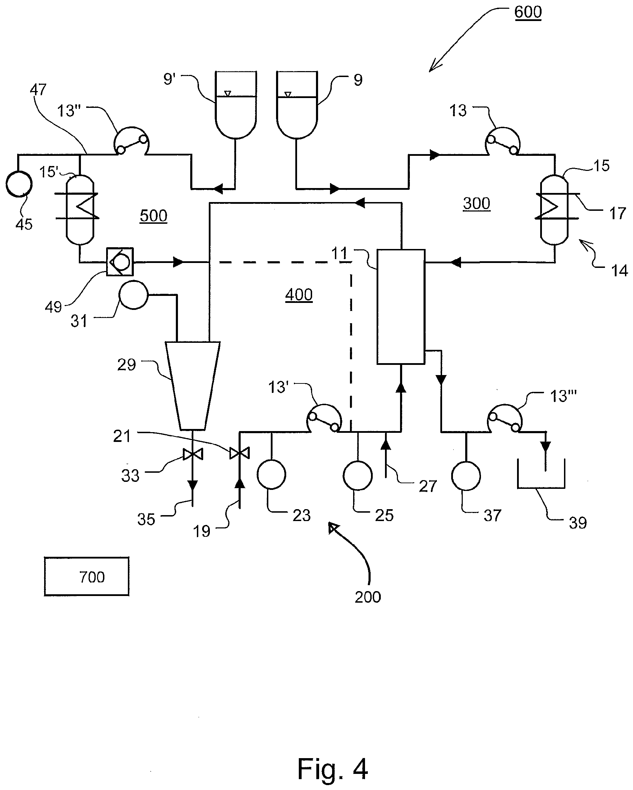

Die Druckbestimmung mittels des Druckaufnehmers 41 hat den zusätzlichen Vorteil, dass Druckanstiege im Heizbeutel 15 erkannt werden können. Letztere können beispielsweise aufgrund einer geschlossenen Klemme entstehen, welche versehentlich auf die Dialysatleitung stromab des Dialysators 11 aufgesetzt bzw. von dieser bei Behandlungsbeginn nicht entfernt wurde. Der Heizbeutel 15 kann so, etwa durch Anhalten der Dialysierflüssigkeitspumpe 13, vor zu hohem Druck und damit vor einer Beschädigung bewahrt werden. Das Anhalten kann automatisch erfolgen, es kann alternativ oder ergänzend aber auch ein entsprechender Alarm ergehen. Ein wie hier beschriebenes Bestimmen des Drucks und die damit verbundenen Vorteile sind auch in anderen Abschnitten des Schlauchsystems 200 möglich bzw. erzielbar, wie dies in Fig. 4 rein exemplarisch für das Substituat-Schlauchsystem 500 mit einem Druckaufnehmer 45 und einem Ventil 49 gezeigt ist. Es wird darauf hingewiesen, dass die in den Figuren gezeigten und/oder vorstehend beschriebenen erfindungsgemäßen Ausgestaltungen keineswegs auf ihre Anwendung auf Schlauchsysteme beschränkt sind, welche in der Dialyse verwendet werden. Jedes anderes Behandlungsverfahren kann ebenfalls von der vorliegenden Erfindung profitieren.

In den Figuren ist eine Zugabe von Substituat in Postdilution (also stromab des Dialysators 11) dargestellt. Die Strichlinie zeigt jeweils an, dass eine Zugabe auch in Prädilution (also stromauf des Dialysators 11) möglich und vorgesehen sein kann.

**Fig. 2** zeigt schematisch eine erfindungsgemäße medizinische Vorrichtung mit einem Schlauchsystem in einer zweiten Ausführungsform.

In Fig. 2 weist das Dialysierflüssigkeits-Schlauchsystem 300 einen Druckaufnehmer 41 auf, welcher in einer Dialysierflüssigkeitsleitung 43 angeordnet ist. Fig. 2 zeigt das rein optionale Ventil 1 der Fig. 1 nicht. Gleichwohl könnte dies auch hier vorgesehen sein.

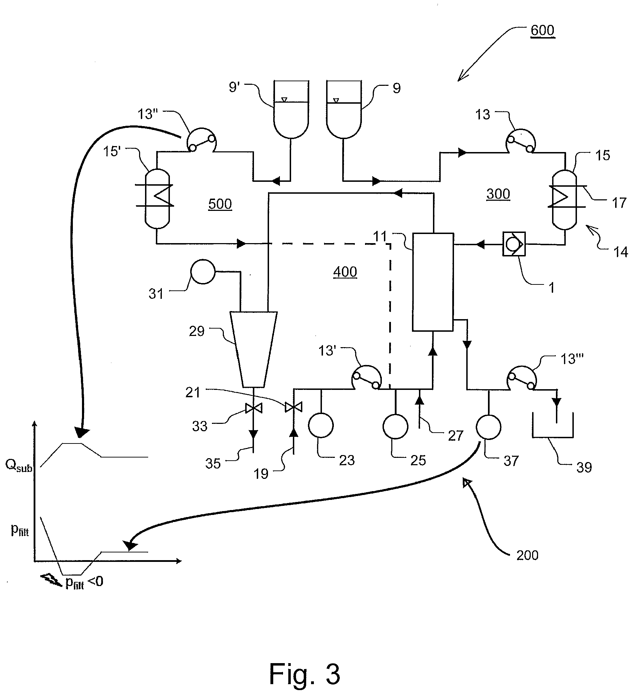

**Fig. 3** zeigt schematisch eine erfindungsgemäße medizinische Vorrichtung mit einem Schlauchsystem in einer dritten Ausführungsform. In Fig. 3 weist das Dialysierflüssigkeits-Schlauchsystem 300 den optionalen Druckaufnehmer 41 nicht auf.

Fig. 3 stellt eine der möglichen Funktionsweisen der Steuer- oder Regelungsvorrichtung 700 bildlich dar. Bei der in Fig. 3 gezeigten Regelung, welche auch als Steuerung ausgestaltet sein kann, wird mittels der Vorrichtung 700 dann, wenn der Drucksensor 37 stromabwärts des Dialysators 11 einen Druck, hier den Filtratdruck p_filt, bestimmt, welcher beispielsweise unter dem Referenzdruck liegt, auf die Substituatpumpe 13" eingewirkt. Die Einwirkung senkt den Substituatfluss Q_sub, beispielsweise bei einer CVV-HDF-Behandlung, bis, oder so dass, der Filtratdruck p_filt wieder über den Referenzdruck ansteigt. Im Beispiel der Fig. 3 entspricht der Referenzdruck dem Umgebungsdruck. Übersteigt der Filtratdruck p_filt den Referenz- oder den Umgebungsdruck wieder, so kollabiert der Heizbeutel 15 nicht (mehr).

Hierzu sei ergänzend erläutert, dass die vorstehend ausgeführte Regelung/Steuerung ausgerichtet ist, um die eingestellte Gesamtgewichtsabnahme, welche sich aus der Bilanz zwischen dem Gewicht des dem Patienten entzogenen Fluids und dem Gewicht des dem Patienten durch Substitution zugeführten Fluids ergibt, unangetastet zu lassen. Droht der Heizbeutel 15 zu kollabieren, so muss die Flussrate der (Filtrat-)Pumpe 13''' reduziert werden, um einem Kollabieren entgegen zu wirken. Infolge dieser Reduzierung wird weniger Filtrat über die Dialysatormembran abfiltriert. Damit entspricht die Rate der Gewichtsabnahme nicht mehr dem, was vom Arzt eingestellt wurde, sie liegt zu niedrig, und dem Patienten wird insgesamt weniger Fluid entzogen, als gewünscht. Letzteres wird nun dadurch kompensiert, dass die Substituatrate mittels der Substituatpumpe 13" ebenfalls entsprechend reduziert wird. Der ausgehend von der eingestellten Ultrafiltrationsrate angestrebte Flüssigkeitshaushalt wird auf diese Weise durch Verringerung von sowohl der Ultrafiltration als auch der Gabe von Substituat sichergestellt. Dies ist in Fig. 3 mittels der Pfeile angedeutet.

**Fig. 4** zeigt schematisch eine erfindungsgemäße medizinische Vorrichtung für die Dialyse mit einem Schlauchsystem in einer vierten Ausführungsform.

In Fig. 4 ist anstelle des Druckaufnehmers 41 in der optionalen Dialysierflüssigkeitsleitung 43 ein ebenfalls rein optionaler Druckaufnehmer 45 einer Substituatleitung 47 stromabwärts der Substituatpumpe 13" vorgesehen. Die Ausführungsform der Fig. 4 weist zudem ein Ventil 49 stromabwärts des Heizbeutels 15' auf. Das Ventil 49 kann in Funktion und Aufbau dem Ventil 1 der Fig. 1 und 3 entsprechen.

Das Vorsehen des Druckaufnehmers 45 erlaubt vorteilhafter Weise auch ein Erkennen eines Überdrucks oder eines unzulässig hohen Drucks im Heizbeutel 15'. Damit kann durch Ergreifen geeigneter Maßnahmen wie Stoppen oder Herabregeln/-steuern der entsprechenden Pumpe ein Platzen des Heizbeutels 15' verhindert werden.

Es wird darauf hingewiesen, dass erfindungsgemäße Ausführungsformen unabhängig voneinander ein Ventil 1, ein Ventil 49, einen Drucksensor 41, einen Druckaufnehmer 45 und/oder einen Druckaufnehmer 37 aufweisen können. Die Steuer- oder Regelvorrichtung 700 ist jeweils geeignet konfiguriert um wenigstens die Pumpen 13, 13" oder 13''' sowie optional die Ventile 1, 49, sofern gezielt mittels eines Aktors beispielsweise durch Öffnen oder Schließen verstellbar, zu beeinflussen mit dem Ziel, eine Veränderung des jeweils überwachten oder ermittelten Drucks in einen Wunschbereich oder jenseits eines Schwellenwertes zu bewirken.

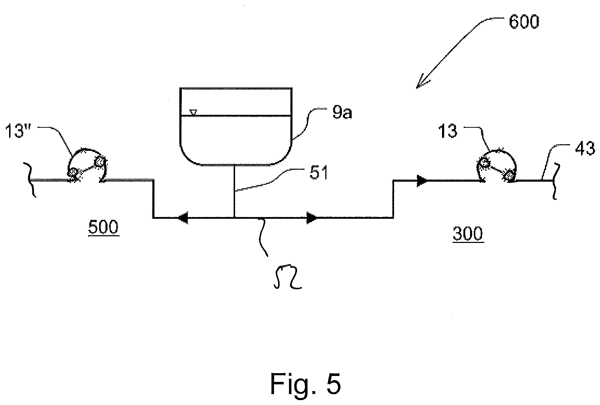

**Fig. 5** zeigt schematisch stark vereinfacht eine erste erfindungsgemäße Weiterbildung eines Ausschnitts der medizinischen Vorrichtung.

In dieser Ausführungsform sind die Behälter 9 und 9' der vorangegangenen Figuren zu einem Behälter 9a vereinigt. Aus dem Behälter 9a werden die Dialysierflüssigkeitspumpe 13 und die Substituatpumpe 13" gemeinsam versorgt, beispielsweise mittels einer gemeinsamen Leitung 51.

Die erfindungsgemäße Ausgestaltung der Fig. 5, welche anders als die in den vorangegangenen Figuren beschriebenen Ausgestaltungen eine gemeinsame Versorgung von Dialysierflüssigkeitspumpe 13 und Substituatpumpe 13" aus nur einer, jedenfalls aber aus einer gemeinsamen Quelle vorsieht, kann jede hierin offenbarte Merkmalskombination oder Ausführungsform ergänzen. Diese Ausgestaltung ist somit kombinierbar mit jeder der hierin gemachten Ausführungsformen und kann dabei die getrennte Zufuhr aus getrennten Quellen ungeachtet aller übrigen Ausgestaltungsdetails ersetzen.

In Fig. 5 ist ferner eine Verbindungsleitung 52 zwischen der Zufuhrleitung der Dialysierflüssigkeitspumpe 13 und der Zufuhrleitung der Substituatpumpe 13" gezeigt.

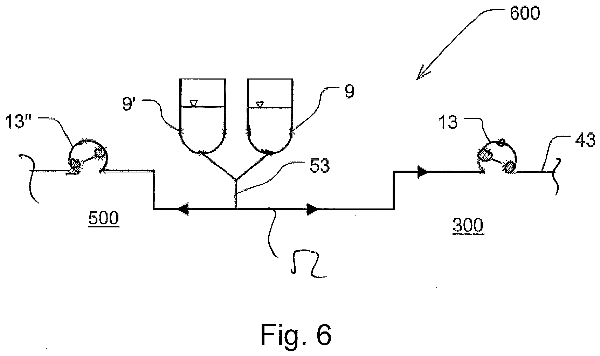

**Fig. 6** zeigt schematisch stark vereinfacht eine zweite erfindungsgemäße Weiterbildung eines Ausschnitts der medizinischen Vorrichtung.

Die erfindungsgemäße Ausgestaltung der Fig. 6, welche anders als die in den vorangegangenen Figuren beschriebenen Ausgestaltungen ein Y-Stück 53 zur gemeinsamen Versorgung von Dialysierflüssigkeitspumpe 13 und Substituatpumpe 13", allerdings aus getrennten Quellen, vorsieht, kann ebenfalls jede hierin offenbarte Merkmalskombination oder Ausführungsform ergänzen. Diese Ausgestaltung ist somit kombinierbar mit jeder der hierin gemachten Ausführungsformen und kann dabei die getrennte Zufuhr aus getrennten Zufuhrleitungen ungeachtet aller übrigen Ausgestaltungsdetails ersetzen.

In den Fig. 5 und 6 stehen die Zufuhrleitungen für Substituat und Dialysierflüssigkeit mittels der Verbindungsleitung 52 miteinander in Fluidverbindung. Auch diese Ausgestaltung kann ebenfalls jede hierin offenbarte Merkmalskombination oder Ausführungsform ergänzen, ungeachtet der übrigen Merkmale.

**Bezugszeichenliste**

| **Bezugszeichen** | **Beschreibung** |
|---|---|
| 200 | Schlauchsystem |
| 300 | Dialysierflüssigkeits-Schlauchsystem |
| 400 | Blutkreislauf |
| 500 | Substituat-Schlauchsystem |
| 600 | Medizinische Vorrichtung |
| 700 | Steuer- oder Regelvorrichtung |
| 1 | Rückschlagventil, Ventil |
| 9,9' | Beutel |
| 9a | Behälter |
| 11 | Dialysator |
| 13 | Dialysierflüssigkeitspumpe |
| 13' | Blutpumpe |
| 13" | Substituatpumpe |
| 13''' | Dialysatpumpe oder Filtratpumpe |
| 14 | Heizvorrichtung |
| 15,15' | Heizbeutel |
| 17 | Heizwendel, Heizstäbe |
| 19 | arterieller Anschluss |
| 21 | Absperrhahn, arteriell |
| 23 | Drucksensor für arteriellen Druck |
| 25 | Drucksensor für Hämofiltrationsdruck oder Vorfilterdruck |
| 27 | Zugabestelle für Heparin |
| 29 | venöse Tropfkammer |
| 31 | Drucksensor für venösen Druck |
| 33 | Absperrhahn, venös |
| 35 | venöser Anschluss |
| 37 | Drucksensor für Filtratdruck |
| 39 | Auffangbehälter |
| 41 | Druckaufnehmer |
| 43 | Dialysierflüssigkeitsleitung |
| 45 | Druckaufnehmer |
| 47 | Substituatleitung |
| 49 | Rückschlagventil, Ventil |
| 51 | gemeinsame Leitung |
| 52 | Verbindungsleitung zwischen den Pumpen |
| 53 | Y-Stück |

## Patentansprüche

1. Medizinische Vorrichtung (600) ausgestaltet als Blutreinigungseinrichtung, aufweisend eine Steuer- oder Regelvorrichtung (700), welche konfiguriert ist zum Durchführen eines Verfahrens zum Steuern oder Kontrollieren des Drucks (p_filt), welcher während der Behandlung des Blut eines Patienten innerhalb eines Heizbeutels (15, 15') eines zur Behandlung verwendeten Schlauchsystems (200) herrscht, wobei das Verfahren die Schritte umfasst:
- Bestimmen des Drucks (p_filt);
- Vergleichen des bestimmten Drucks (p_filt) mit einem Referenzdruck oder Ermitteln seiner Lage bezogen auf einen Referenzdruckbereich;
- Ändern wenigstens eines Behandlungsparameters der Behandlung des Patienten oder Vorschlagen einer Korrektur oder Änderung des wenigstens einen Behandlungsparameters falls der bestimmte Druck (p_filt) unterhalb bzw. oberhalb des Referenzdrucks oder außerhalb des Referenzdruckbereichs liegt, wobei der Behandlungsparameter der Behandlung ein Substitutionsfluss oder eine Ultrafiltrationsrate ist.

2. Medizinische Vorrichtung (600) nach Anspruch 1, wobei bei dem Verfahren der Substitutionsfluss soweit reduziert wird, dass oder bis der Druck (p_filt) wieder oberhalb bzw. unterhalb des Referenzdrucks oder innerhalb des Referenzdruckbereichs liegt oder gelangt.

3. Medizinische Vorrichtung (600) nach einem der vorangegangenen Ansprüche, wobei bei dem Verfahren der Referenzdruck der Umgebungsdruck ist.

4. Medizinische Vorrichtung (600) nach einem der vorangegangenen Ansprüche, wobei bei dem Verfahren der Druck (p_filt) mittels eines Druckaufnehmers (41) bestimmt wird, welcher in einer Dialysierflüssigkeitsleitung (43) des Schlauchsystems (200) stromabwärts einer Dialysierflüssigkeitspumpe (13) angeordnet ist.

5. Medizinische Vorrichtung (600) nach einem der vorangegangenen Ansprüche, wobei bei dem Verfahren der Druck (p_filt) mittels einer stromaufwärts eines Dialysators (11) und stromabwärts der Heizvorrichtung (14) angeordneten Vorrichtung beeinflusst wird, welche aus einer Gruppe ausgewählt ist, welche ein Ventil (1, 49), eine Drossel und eine Blende aufweist.

6. Medizinische Vorrichtung (600) nach einem der vorangegangenen Ansprüche, wobei bei dem Verfahren der Druck (p_filt) mittels eines Druckaufnehmers (45) bestimmt wird, welcher in einer Substituatleitung (47) des Schlauchsystems (200) stromabwärts einer Substituatpumpe (13") angeordnet ist.

7. Medizinische Vorrichtung (600) nach einem der vorangegangenen Ansprüche, wobei der Heizbeutel (15, 15') Dialysierflüssigkeit oder Substituat enthält.

8. Medizinische Vorrichtung (600) nach einem der vorangegangenen Ansprüche, ausgestaltet als Dialysevorrichtung.

## Claims

1. A medical apparatus (600) designed as a blood purification device, comprising a control or regulating apparatus (700) configured to perform a method of controlling or checking the pressure (p_filt), which prevails during the treatment of a patient's blood within a heating bag (15, 15') of a tubing system (200) used for the treatment, the method comprising the steps of:
- determining the pressure (p_filt);
- comparing the determined pressure (p_filt) with a reference pressure or determining its position relative to a reference pressure range;
- modifying at least one treatment parameter of the treatment of the patient or proposing a correction or modification of the at least one treatment parameter if the determined pressure (p_filt) lies below, respectively above the reference pressure or outside the reference pressure range, wherein the treatment parameter of the treatment is a substitution flow or an ultrafiltration rate.

2. The medical apparatus (600) according to claim 1, wherein in the method, the substitution flow is reduced to such an extent that or until the pressure (p_filt) lies or reaches again above, respectively below the reference pressure or within the reference pressure range.

3. The medical apparatus (600) according to anyone of the preceding claims, wherein in the method the reference pressure is the ambient pressure.

4. The medical apparatus (600) according to anyone of the preceding claims, wherein in the method the pressure (p_filt) is determined by means of a pressure sensor (41) arranged in a dialysis fluid line (43) of the tubing system (200) downstream of a dialysis fluid pump (13).

5. The medical apparatus (600) according to anyone of the preceding claims, wherein in the method the pressure (p_filt) is influenced by means of an apparatus arranged upstream of a dialyzer (11) and downstream of a heating apparatus (14), the apparatus being selected from a group comprising a valve (1, 49), a throttle and a diaphragm.

6. The medical apparatus (600) according to anyone of the preceding claims, wherein in the method the pressure (p_filt) is determined by means of a pressure sensor (45) arranged in a substitute line (47) of the tubing system (200) downstream of a substitute pump (13").

7. The medical apparatus (600) according to anyone of the preceding claims, wherein the heating bag (15, 15') contains dialysis fluid or substitute.

8. The medical apparatus (600) according to anyone of the preceding claims, designed as a dialysis apparatus.

## Revendications

1. Un appareil médical (600) conçu comme un dispositif de purification du sang, comprenant un appareil de commande ou de régulation (700) configuré pour exécuter un procédé de commande ou de contrôle de la pression (p_filt) régnant pendant le traitement du sang d'un patient dans une poche chauffante (15, 15') d'un système de tuyaux (200) utilisé pour le traitement, le procédé comprenant les étapes consistant à:
- déterminer la pression (p_filt);
- comparer la pression déterminée (p_filt) avec une pression de référence ou déterminer sa position par rapport à une plage de pression de référence;
- modifier au moins un paramètre de traitement du traitement du patient ou proposer une correction ou une modification d'au moins un paramètre de traitement si la pression déterminée (p_filt) se situe en dessous, respectivement au-dessus de la pression de référence ou en dehors de la plage de pression de référence,
où le paramètre de traitement du traitement est un flux de substitution ou un taux d'ultrafiltration.

2. L'appareil médical (600) selon la première revendication, où dans le procédé, le flux de substitution est réduit à un point tel que ou jusqu'à ce que la pression (p_filt) se situe ou parvienne à nouveau au-dessus, respectivement en dessous de la pression de référence ou à l'intérieur de la plage de pression de référence.

3. L'appareil médical (600) selon l'une quelconque des revendications précédentes, où dans le procédé, la pression de référence est la pression ambiante.

4. L'appareil médical (600) selon l'une quelconque des revendications précédentes, où dans le procédé, la pression (p_filt) est déterminée au moyen d'un capteur de pression (41) agencé dans un conduit de liquide de dialyse (43) du système de tuyaux (200) en aval d'une pompe de liquide de dialyse (13).

5. L'appareil médical (600) selon l'une quelconque des revendications précédentes, où dans le procédé, la pression (p_filt) est influencée au moyen d'un appareil agencé en amont d'un dialyseur (11) et en aval de l'appareil de chauffage (14), sélectionné à partir d'un groupe comprenant une vanne (1, 49), un étrangleur et un diaphragme.

6. L'appareil médical (600) selon l'une quelconque des revendications précédentes, où dans le procédé, la pression (p_filt) est déterminée au moyen d'un capteur de pression (45) agencé dans un conduit de substitut (47) du système de tuyaux (200) en aval d'une pompe à substitut (13").

7. L'appareil médical (600) selon l'une quelconque des revendications précédentes, où la poche chauffante (15, 15') contient du liquide de dialyse ou du substitut.

8. L'appareil médical (600) selon l'une quelconque des revendications précédentes, conçu comme un appareil de dialyse.
